(19) Europäisches Patentamt
European Patent Office
Office européen des brevets

(11) Publication number: 0 381 531 A2

# (12) EUROPEAN PATENT APPLICATION

(21) Application number: 90301144.3

(22) Date of filing: 02.02.90

(51) Int. Cl.5: C07D 473/00, C07F 9/6564, A61K 31/52

(30) Priority: 02.02.89 US 305265

(43) Date of publication of application: 08.08.90 Bulletin 90/32

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Hannah, John
155 Idlebrook Lane
Matawan, New Jersey 07747(US)
Inventor: Tolman, Richard L.
29 Upper Warren Way
Warren, New Jersey 07060(US)

(74) Representative: Hesketh, Alan, Dr. et al
European Patent Department Merck & Co., Inc. Terlings Park Eastwick Road
Harlow Essex, CM20 2QR(GB)

## (54) Antiviral acyclonucleosides and acyclonucleotides.

(57) Disclosed are compounds of the formula:

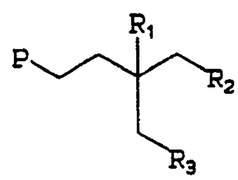

wherein B is related to one of the heterocyclic bases found in nucleic acid (DNA, RNA) such as a purine, a substituted purine, a pyrimidine or a substituted pyrimidine wherein the substituents on the purine or pyrimidine are selected from amino, hydroxyl, halogen, thio or alkylthiol wherein the alkyl moiety of the alkylthiol has 1 to 6 carbons; $R^1$ is fluorine, chlorine, hydroxy, alkoxy having 1 to 6 carbons or a phosphate group having the formula:

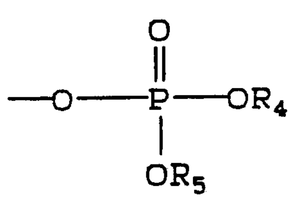

wherein $R^4$ and $R^5$ are pharmaceutically acceptable cations; $R^2$ and $R^3$ are independently hydroxy, alkoxy having 1 to 6 carbons or a phosphate group having the formula:

EP 0 381 531 A2

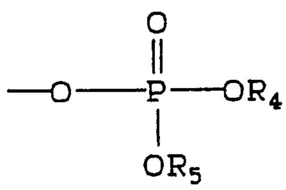

wherein $R^4$ and $R^5$ hydrogen or are pharmaceutically acceptable cations; or two of $R^1$, $R^2$ and $R^3$ taken together form a phosphate group having the formula:

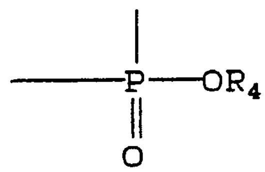

wherein $R^4$ is hydrogen or a pharmaceutically acceptable cation. The compounds have antiviral activity. They are particularly effective against the herpes group of viruses with special utility against cytomegalovirus (CMV) and varicella-zoster virus (VZV), and against retroviruses such as HTLV-III.

# ANTIVIRAL ACYCLONUCLEOSIDES ACYCLONUCLEOTIDES

## BACKGROUND OF TEE INVENTION

The present invention relates to purine and pyrimidine acyclonucleosides and acyclonucleotides. These compounds have antiviral activity. The compounds are particularly effective against the herpes group of viruses, with special utility against cytomegalovirus (CMV) and varicella-zoster virus (VZV) and against retroviruses such as HTLV III. The present invention also relates to processes for preparing said compounds, pharmaceutical compositions comprising said compounds and the treatment of viral infections in mammals with said compounds.

Other purine and pyrimidine derivatives claiming antiviral activity have previously been disclosed. Pandit et al., in Synthetic Communications 2(6), 345-351 (1972) provides a method of synthesis of 4-hydroxy-3-(hydroxymethyl) butyl derivatives of nucleobases. Other references by Hagberg et al. (United States Patent 4,495,190), Verheydeh et al. (United States Patent 4,565,868), Prisbe et al. (United States Patent 4,590,269), MacCoss et al. (United States Patent 4,579,849), and Jarvest et al. (EPO Published Application 141927) relate to guanine and purine derivatives which have antiviral activity.

Continued interest in developing effective anti-herpes simplex agents and the increasing necessity to develop effective agents for the treatment and or prevention of DNA viruses, such as Varicella- Zoster, cytomegalovirus, Epstein-Barr virus and retroviruses such as HTLV III has resulted in the development of the compounds of the present invention.

## DESCRIPTION OF THE INVENTION

The compounds of the present invention may be represented by the formula:

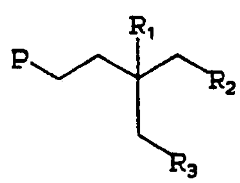

and the pharmaceutically acceptable salts thereof wherein B is related to one of the heterocyclic bases found in nucleic acid (DNA,RNA) such as related to one of the heterocyclic bases found in nucleic acid (DNA,RNA) such as a purine, a substituted purine, a pyrimidine or a substituted pyrimidine wherein the substituents on the purine or pyrimidine are selected from amino, hydroxyl, halogen, thio or alkylthiol wherein the alkyl moiety of the alkylthiol has 1 to 6 carbons; $R^1$ is fluorine, chlorine, hydroxy, alkoxy having 1 to 6 carbons or a phosphate group having the formula:

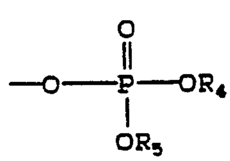

wherein $R^4$ and $R^5$ are hydrogen or pharmaceutically acceptable cations; $R^2$ and $R^3$ are independently hydroxy, alkoxy having 1 to 6 carbons or a phosphate group having the formula:

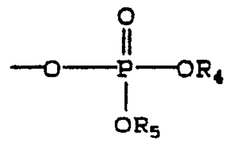

or two of $R^1$, $R^2$ and $R^3$ taken together form a phosphate group having the formula:

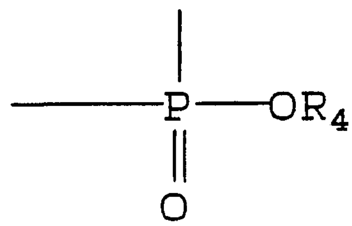

wherein $R^4$ is hydrogen or a pharmaceutically acceptable cation.

The reaction scheme for preparing the 9-[(3-substituted-3-hydroxymethyl-4-hydroxy)butyl]guanine compounds of the present invention is as follows:

SCHEME I

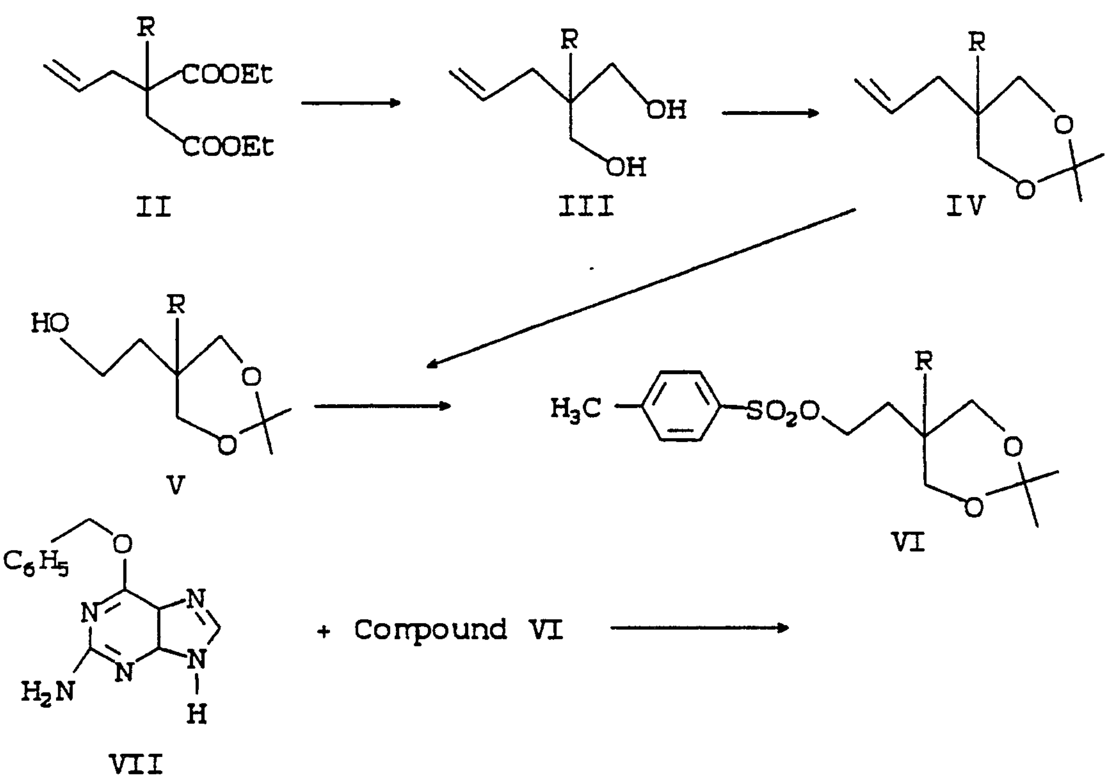


II III IV V VI VII

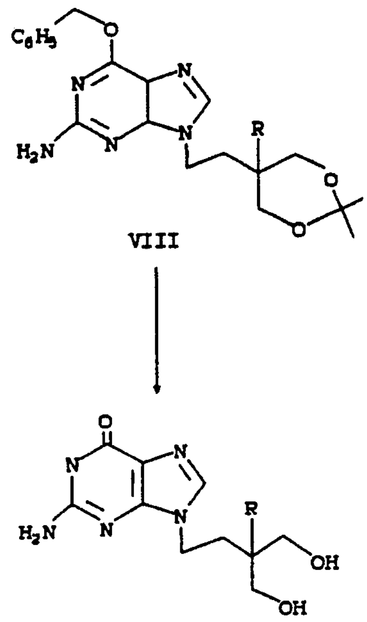

VIII

IX

The diester compound II is reduced to the diol compound III. The diol compound III is then selectively protected by the formation of the acetonide IV, followed by ozonolysis and reduction to form compound V. Compound V is then converted to its tosyl derivative VI, which is then reacted with a substituted guanine VII which results in alkylation of VII to form VIII. Compound VIII is then subjected to hydrolysis to remove the acetonide protecting group and convert the the 6-benzyloxy on the guanine to a 6-oxo functionality.

While the reaction scheme represented above employs guanine as the nucleic acid, other purine, substituted purines, pyrimidines and substituted pyrimidines may also be used, and are considered part of the present invention. Purines and substituted purines, pyrimidines and substituted pyrimidines are defined as the common heterocyclic bases of nucleic acids and include but are not limited to adenine, guanine, cytosine, thymine and uracil.

The compounds of the present invention also include their cyclic monophosphate derivatives. These cyclic monophosphate derivatives may be formed by a variety of methods which include direct phosphorylation of compound IX using orthochlorophenylphosphorodichloridate followed by deprotection by standard hydrogenation conditions, for example hydrogenation with hydrogen gas in the presence of a noble metal catalyst such as palladium or platinum.

While purines, substituted purines, pyrimidines substituted pyrimidines that are represented by B, as defined above, may be attached to the sidechain from various positions on the purines, substituted purines, pyrimidines, and substituted pyrimidines, the preferred position for the purines is the 9-position and the preferred position for the pyrimidines is the 1-position.

The term "pharmaceutically acceptable cation" refers to those cations which possess the biological effectiveness and properties of the free compound and which are not biologically or otherwise undesirable. Examples of these cations include ions of sodium, potassium, lithium, calcium, magnesium, ammonium, as well as any other acceptable cation known in the art.

The following compounds are representative of the compounds of the present invention:

9-[(4-hydroxy-3-hydroxymethyl-3-methoxy)butyl]guanine;
9-[(4-hydroxy-3-fluoro-3-hydroxymethyl)butyl]guanine;
9-[(3,4-dihydroxy-3-hydroxymethyl)butyl]guanine;
9-[(4-hydroxy-3-hydroxymethyl-3-methoxy)butyl]guanine cyclic monophosphate;
9-[(4-hydroxy-3-fluoro-3-hydroxymethyl)butyl]guanine cyclic monophosphate;
9-[(3,4-dihydroxy-3-hydroxymethyl)butyl]guanine cyclic monophosphate;
9-[(4-hydroxy-3-hydroxymethyl-3-methoxy)butyl]adenine;
9-[(4-hydroxy-3-fluoro-3-hydroxymethyl)butyl]adenine;
9-[(3,4-dihydroxy-3-hydroxymethyl)butyl]adenine;
9-[(4-hydroxy-3-hydroxymethyl-3-methoxy)butyl]adenine cyclic monophosphate;

9-[(4-hydroxy-3-fluoro-3-hydroxymethyl)butyl]adenine cyclic monophosphate;
9-[(3,4-dihydroxy-3-hydroxymethyl)butyl]adenine cyclic monophosphate;
1-[(4-hydroxy-3-hydroxymethyl-3-methoxy)butyl]cytosine;
1-[(4-hydroxy-3-fluoro-3-hydroxymethyl)butyl]cytosine;
1-[(3,4-dihydroxy-3-hydroxymethyl)butyl]cytosine;
1-[(4-hydroxy-3-hydroxymethyl-3-methoxy)butyl]cytosine cyclic monophosphate;
1-[(4-hydroxy-3-fluoro-3-hydroxymethyl)butyl]cytosine cyclic monophosphate;
1-[(3,4-dihydroxy-3-hydroxymethyl)butyl]cytosine cyclic monosphosphate.

Preferred compounds of the present invention are compounds of formula I wherein B is adenine, guanine, cytosine, thymine or uracil, $R^1$ is hydroxy, alkoxy having 1 to 6 carbons, fluorine or chlorine, $R^2$ and $R^3$ are hydrogen or taken together are $-OPO_2O-$. Especially preferred compounds are where B is guanine or cytosine and $R^1$, $R^2$ and $R^3$ are as defined above.

In another aspect of the invention there is provided a pharmaceutical composition or preparation comprising a compound of the formula:

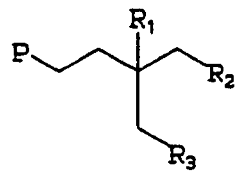

wherein B, $R^1$, $R^2$ and $R^3$ are hereinbefore defined; or a pharmaceutically acceptable salt thereof; together with a pharmaceutically acceptable carrier therefor. In a particular aspect the pharmaceutical composition comprises a compound of the formula:

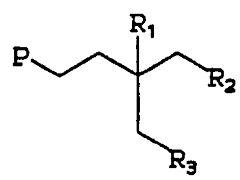

wherein B, $R^1$, $R^2$ and $R^3$ are hereinbefore defined in effective unit dosage form.

As used herein the term "effective unit dosage" or "effective unit dose" is denoted to mean a predetermined antiviral amount sufficient to be effective against the viral organisms in vivo. Pharmaceutically acceptable carriers are materials useful for the purpose of administering the medicament, and may be solid, liquid or gaseous materials, which are compatible with the active ingredients.

These pharmaceutical compositions may be given parenterally, orally, used as a suppository or pessary, applied topically as an ointment, cream, aerosol, powder, or given as eye or nose drops, etc., depending on whether the preparation is used to treat internal or external viral infections.

For internal infections the compositions are administered orally or parenterally at dose levels calculated as the free base, of about 0.1 to 250 mg per kg, preferably 1.0 to 50 mg per kg of mammal body weight, and are used in man in a unit dosage form, administered, for example, three or four times a day, in the amount of 1 to 250 mg per unit dose.

For oral administration, fine powders or granules may contain diluting, dispersing and/or surface active agents, and may be formulated in a draught, in water or in a syrup; in capsules or sachets in the dry state or in a non-aqueous solution or suspension, suspending agents may be included; in tablets, wherein binders and lubricants may be included; or in a suspension in water or a syrup. Where desirable or necessary, flavoring, preserving, suspending, thickening or emulsifying agents may be included. Tablets or granules are preferred, and these may be coated.

For parenteral administration or for administration as drops, as for eye infections, the compounds may be formulated in aqueous solution in a concentration of from about 0.1 to 10%, more preferably 0.1 to 7%, most preferably 0.2% w/v. The solution may contain antioxidants, buffers, etc.

Alternatively for infections of the eye, or other external tissues, e.g. mouth and skin, the compositions are preferably applied to the infected part of the body of the patient as a topical ointment or cream. The

compounds may be presented in an ointment, for instance, with a water soluble ointment base, or in a cream, for instance with an oil in water cream base, in a concentration of from about 0.1 to 10%, preferably 0.1 to 7% most preferably 1% w/v.

The following non-limiting Examples illustrate the preparation of compounds and compositions of the present invention. All temperatures are in ° C.

## EXAMPLE 1

### 9-[(4-hydroxy-3-hydroxymethyl-3-methoxy)butyl]guanine

#### Step A:

Clean sodium (9.74 g, 0.42 mole) was added to anhydrous EtOH (124 ml) and the mixture was boiled under reflux to give a clear solution. Ethyl methoxyacetate (50.0 g, 0.42 mole) and diethyl carbonate (350 ml, 2.89 mole) were added and the solution was slowly distilled for 1 hour 30 minutes, removing approximately 210 ml. The reaction mixture was now a slurry of sodium salt at 120°. The mixture was cooled to 23° and filtered. The solids were washed with benzene, dried at 25°/0.25 mm, and obtained as a tan powder (60.75 g). All of it was suspended in benzene (200 ml). Crushed ice (100 g) was added followed by aq. 4N $H_2SO_4$ to pH 2. The organic phase was washed with saturated aq NaCl, then dried over $MgSO_4$, filtered and evaporated to give an oil which was distilled at reduced pressure: 33.87 g, bp 73-76°/0.4 mm, $n_D^{22°}$ 1.4222 (lit.* bp 95°/3 mm $n_D^{22°}$ 1.4229). TLC (silica GF, hexane/50% EtOAc) showed two products, $R_f$ 0.68 and 0.84, by $I_2$ staining. Both UV and $Ce(SO_4)_2$ charring detected only the more polar component. NMR ($CDCl_3$) clearly showed the product to be a mixture, a portion 29.10 g) of which was separated by chromatography over silica. Hexane/15% EtOAc eluted the less polar component (18.25 g) and EtOAc the more polar component (9.43 g). Each was distilled at reduced pressure to give colorless oils. The less polar product was diethyl methoxymalonate (17.38 g), bp 69°/0.2 mm, $n_D^{22°}$ 1.4175. NMR ($CDCl_3$) δ 1.31 (t, 6 H, J = 7 Hz $[CH_3]_2$); 3.52 (δ, 3 H, $OCH_3$); 4.29 (q, 4 H, J = 7 Hz, $[OCH_2]_2$), 4.41 (δ, 1 H, CH). Anal. calcd. for $C_8H_{14}O_5$: C, 50.52; H, 7.42. Found: C, 50.40; H, 7.16. The more polar product was ethyl 2,4-dimethoxyacetoacetate (7.99 g), bp 73°/0.2 mm, $n_D^{23°}$ 1.4297. NMR ($CDCl_3$), δ 1.32 (t, 3 H, J = 7 Hz, $CH_3$); 3.43 (s, 3 H, $OCH_3$); 3.49 (s 3 H, $OCH_3$); 4.31 (q, 2 H, J = 7 Hz, $OCH_2$); 4.35 (s, 2 H, $CH_2$); 4.46 (s, 1 H, CH). Anal. calcd. for $C_8H_{14}O_5$: C, 50.52; H, 7.42. Found: C, 50.58; H, 7.59.

2.5 M nBuLi (35.8 ml, 89.4 mmole) in hexane was added dropwise in 10 minutes by hypodermic through a rubber septum to a magnetically stirred solution of diethyl methoxymalonate (17.0 g, 89.4 mmole) in anhydrous tert BuOH (85 ml) at 22°. The very vigorous reaction was controlled by cooling in an ice bath. With the ice bath still in place, redistilled allyl bromide (8.51 ml, 98.3 mmole) was added in 4 minutes in the same manner. After 10 more minutes the ice bath was removed and the mixture was allowed to stand at room temperature for 1 hour becoming a crystalline slurry. The mixture was partitioned between ether (500 ml) and water (100 ml), dried over $MgSO_4$, filtered and evaporated at 70°/100 mm to give a pale yellow oil (19.70 g). TLC (silica GF, hexane/25% EtOAc, $Ce(SO_4)_2$, single spot $R_f$ 0.60). A portion was distilled at reduced pressure: bp 110°/3.5 mm, $n_D^{26°}$ 1.4310. NMR ($CDCl_3$) δ 1.30 (t, 6 H, J = 7 Hz, $[CH_3]_2$; 2.84 (m, 2 H, $CH_2$); 4.27 (q, 4 H, J = 7 Hz, $[OCH_2]_2$); 5.14 (m, 2 H, $=CO_2$); 5.75 (m, 1 H, =CH-). Anal. calcd. for $C_{11}H_{18}O_5$: C, 57.38; H, 7.88. Found: C, 57.16; H, 7.77.

#### Step B:

The product of Step A, diethyl allylmethoxymalonate (19.7 g, 85.5 mmole) was added in a rapid stream in 30 minutes to a mechanically stirred 1.0 M solution of lithium aluminum hydride in THF (Aldrich) (530 ml). The vigorously exothermic reaction was controlled by a reflux condenser and the occasional use of an ice bath. The completed reaction solution was allowed to cool to room temperature for 30 minutes, then the complex was decomposed by the dropwise addition of saturated aqueous NaCl (400 ml) (ice bath) with very vigorous stirring to disperse an initially gelatinous mass to a final granular precipitate. After adding $MgSO_4$ - (100 g), the mixture was cooled to 25° and filtered. The insolubles were washed with ether (2x100 ml) and the combined organic solutions were evaporated to a residue which was distilled to give a colorless oil (10.0

g), bp 88°/0.1 mm, $n_D^{24°}$ 1.4706. NMR ($CDCl_3$) δ 2.27 (d, 2 H, J = 8 Hz, $CH_2$); 3.21 (br s, 2 H, $[OH]_2$); 3.34 (s, 3H, $OCO_3$); 3.64 (d, 2 H, J = 12 Hz, $[CH_AO]_2$); 3.72 (d, 2 H, J = 12 Hz, $[CH_BO]_2$); 5.14 (m, 2 H, $=CH_2$); 5.78 (ddt, 1 H, J = 8, 9 and 17 Hz, =CH-). Anal. calcd. for $C_7H_{14}O_3$•0.30 $H_2O$: C, 55.46; H, 9.71. Found: C, 55.44; H, 9.73.

Step C:

p-Toluenesulphonic acid monohydrate (88 mg) was added to a solution of the product of Step 8, 2-allyl-2-methoxypropane-1,3-diol (8.88 g) in a 10 fold excess of freshly distilled 2,2-dimethoxypropane (72 ml) at 23°. After 1 hour the solution was shaken with fresh aq 5% $NaHCO_3$ (5 ml), then saturated aq. NaCl (20 ml) was added and the mixture was again shaken. The lower aqueous layer was run off and the organic phase was washed with more saturated aq. NaCl (20 ml); dried over $MgSO_4$; filtered; and evaporated at 60°/120 mm to give a colorless oil. Attempted distillation of this product at pressures from 5 to 120 mm was abandoned because of extensive, persistent frothing. Distillation at atmospheric pressure however, gave a colorless oil which was then easily redistilled in high yield to furnish 5-allyl-2,2-dimethyl-5-methoxy-1,3-dioxane (9.17 g), bp 76-77°/4.5 mm, $n_D^{23°}$ 1.4430. NMR ($CDCl_3$) δ 1.40 (s, 3 H, $CH_3$); 1.43 (s, 3 H $CH_3$); 2.36 (m, 2 H, $CH_2$); 3.33 (s, 3 H, $OCH_3$); 3.69 (d, 2 H, J = 11.9 Hz, $[CH_AO]_2$); 3.76 (d, 2 H, J = 11.9 Hz, $[CH_BO]_2$); 5.15 (m, 2 H, $=CH_2$); 5.80 (m, 1 H, =CH-). Anal calcd. for ($C_{10}H_{18}O_3$) C, 64.49; H, 9.74. Found = C, 64.17; H, 9.60.

Step D:

5-allyl-2,2-dimethyl-5-methoxy-1,3-dioxane (8.87 g, 47.6 mmole) was dissolved in acetonitrile (140 ml) and the solution was cooled to -15° in a MeOH/ice bath. A stream of $O_2/O_3$ from a Welsbach T816 generator (approx. 0.7 mmole $O_3$/min) was bubbled through the solution via a fine porosity gas inlet tube, and the disappearance of starting material ($R_f$ 0.67) was monitored by TLC (silica GF, hexane/25% EtOAc, $Ce(SO_4)_2$). Passage of $O_3$ was stopped after 65 minutes. To the solution of ozonide was added an excess of 1.0 M lithium aluminum hydride in THF (46 ml) at a rate not to exceed a reaction temperature of 20°, with stirring and cooling in MeOH/ice; time required was 15 minutes. After 10 minutes at 22° the reaction complex was decomposed by the dropwise addition of saturated aqueous NaCl (46 ml) with cooling and very vigorous mechanical stirring. $MgSO_4$ (10 g) was added and the mixture was filtered. The solids were washed with ether (2x50 ml) and the combined organic solutions were evaporated to a residue which was chromatographed over silica gel (EtOAc, 0-5% MeOH, with extensive tailing) to give single spot material (6.79 g) (TLC, silica GF, EtOAc, $Ce(SO_4)_2$, $R_f$ 0.39) which was distilled as a colorless oil (5.09 g) bp 84°/0.16 mm, $n_D^{25°}$ 1.4554. NMR ($CDCl_3$) δ 1.40 (s, 3 H, $CH_3$); 1.44 (s, 3 H, $CH_3$); 1.81 (t, 2 H, J = 6.1 Hz, $CH_2$); 2.75 (br s, 1 H, OH); 3.38 (s, 3 H, $OCH_3$); 3.77 (t, 2 H, J = 6.0 Hz, $OCH_2$); 3.75 (d, 2 H, J = 12.4 Hz, $[CH_AO]_2$); 3.88 (d, 2 H, J = 12.4 Hz, $[CH_BO]_2$). Anal. calcd. for $C_9H_{18}O_4$: C, 56.82; H, 9.54. Found: C, 56.72; H, 9.49.

Step E:

A solution of the product of Step D, 2, 2-dimethyl-5-(2-hydroxyethyl)-5-methoxy-1,3-dioxane (1.00 g, 5.3 mmole) in sieve-dried pyridine (6.5 ml) was cooled in an ice bath, and with magnetic stirring, recrystallized p-toluenesulphonylchloride (1.00 g, 5.3 mmole) was added in portions in 3 minutes. After 10 more minutes, the ice bath was removed and the reaction mixture was left at room temperature for 2 hours. The mixture was then partitioned between ether (60 ml) and water (20 ml). The organic phase was washed with fresh aqueous 5% $NaHCO_3$ (10 ml), with water (10 ml), dried ($MgSO_4$), filtered and evaporated. The crude product was shaken with petroleum ether/ether, 1:1 (25+10 ml) decanting the clear supernatant layer from traces of orange gum. The combined extracts were evaporated to give a colorless, viscous oil (1.71 g). TLC single spot (silica GF, hexane/50% EtOAc, $Ce(SO_4)_2$, $R_f$ 0.59). NMR ($CDCl_3$) δ 1.36 (s, 3 H, $CH_3$); 1.39 (s, 3 H, $CH_3$); 1.93 (t, 2 H, J = 6.9 Hz, $CH_2$); 2.45 (s, 3 H, $CH_3$, $TsCH_3$); 3.25 (s, 3 H, $OCH_3$); 3.63 (d, 2 H, J = 12.1 Hz, $[CH_AO]_2$); 3.74 (d, 2 H, J = 12.1 Hz, $[CH_BO]_2$); 4.13 (t, 2 H, J = 6.9 Hz, $OCH_2$); 7.36 and 7.80 (ABq, 4 H, J = 8.2 Hz, Ar).

Step F:

57% Sodium hydride oil dispersion (0.38 g, 9.0 mmol) was added to a magnetically stirred solution of 2-amino-6-benzyloxypurine (1.43 g, 5.9 mmol) in sieve-dried DMF (25 ml) at 22° under dry $N_2$. When effervescence ceased (20 minutes), a solution of the product of Step E, 2,2-dimethyl-5-methoxy-5-(2-p-toluenesulphonyloxyethyl)-1,3-dioxane (1.70 g, 4.9 mmol) in sieve dried DMF (5 ml) was added and stirring was continued overnight (15 hours). The reaction mixture was then partitioned between EtOAc (200 ml) and water (50 ml). The organic layer was washed with more water (2x25 ml), dried ($MgSO_4$), filtered and evaporated at 60°/0.5 mm to give a sticky crystalline residue (2.24 g) which was chromatographed in $CHCl_3/MeOH/H_2O$, 95:5:0.5, over a 4.2 x 36.5 column of silica gel (200 g), collecting 10 ml fractions which were monitored by TLC (silica GF, $CHCl_3/MeOH/H_2O$, 95:5:0.5, $Ce(SO_4)_2$). Fractions 52-86 were combined, evaporated, and the residue was recrystallized twice from EtOAc to give colorless matted needles (748 mg), mp 174-175°. TLC, single spot $R_f$ 0.40 (silica GF, $CHCl_3/MeOH/H_2O$, 90:10:1, $Ce(SO_4)_2$. UV (MeOH) $\lambda_{max}$ 210 nm ($\epsilon$ 26,960), 249 nm ($\epsilon$ 8,870), 283 nm ($\epsilon$ 10,560). NMR ($CDCl_3$) $\delta$ 1.40 (s, 3 H, $CH_3$); 1.44 (s, 3 H, $CH_3$); 2.12 (m, 2 H, $CH_2$); 3.36 (s, 3 H, $OCH_3$); 3.70 (d, 2 H, J = 12.1 Hz, $[CH_AO]_2$); 3.82 (d, 2 H, J = 12.1 Hz, $[CH_BO]_2$); 4.12 (m, 2 H, $NCH_2$); 4.86 (br s, 2 H, $NH_2$); 5.57 (s, 2 H, $OCH_2$); 7.32-7.54 (m, 5 H, Ar); 7.62 (s, 1 H, $C_8$-H). Anal. calcd. for $C_{21}H_{27}N_5O_4$: C, 61.00; H, 6.58; N, 16.94. Found: C, 61.02; H, 6.51; N, 17.04.

Step G:

The product of Step F, 2-amino-6-benzyloxy-9-[2-(2,2-dimethyl-5-methoxy-1,3-dioxan-5-yl)ethyl]-9H-purine (600 mg, 1.45 mmol) was dissolved in hot MeOH (12 ml) and treated with aq. NHCL (2.9 ml). The clear solution was boiled on the steam bath for 10 minutes, removing most of the MeOH. The cold solution was filtered, with water washing (3x1 ml), and the combined aqueous filtrates were neutralized by adding aq. 2.5 N NaOH to pH 7.0. A bulky colorless precipitate was formed, and was filtered off, washed with water (1 ml); EtOH (1 ml); dried at 23°/0.5 mm and obtained as a colorless crystalline powder (397 mg), mp 278-283° d 2-amino-1,9-dihydro-9-(4-hydroxy-3-hydroxy-methyl-3-methoxy)butyl-6-oxo-6H-purine, also known as 9-[(4-hydroxy-3-hydroxymethyl-3-methoxy)butyl]guanine (397 mg), mp 278-283° d. Recrystallization of a portion (53 mg) twice from water gave colorless microprisms (31 mg) mp 282-284° d. UV (aq. 0.01 N NaOH) $\lambda_{max}$ 213 nm ($\epsilon$ 22,560) 255 infl. nm ($\epsilon$ 9,730), 268 nm ($\epsilon$ 10,490). NMR ($D_2O$) $\delta$ 2.06 (t, 2 H, J = 7.8 Hz, $CH_2$); 3.25 (s, 3 H, $OCH_3$); 3.63 (d, 2 H, J = 12.2 Hz, $[CH_A])_2$); 3.68 (d, 2 H, J = 12.2 Hz, $[CH_B])_2$); 4.18 (t, 2 H, J = 7.8 Hz, $NCH_2$); 7.85 (s, 1 H, $C_8$-H). Anal. calcd. for $C_{11}H_{17}N_5O_4$: C, 46.63; H, 6.05; N, 24.72. Found: C, 46.87; H, 6.22; N, 24.56.

## EXAMPLE 2

9-[(4-hydroxy-3-fluoro-3-hydroxymethyl)butyl]guanine

Step A:

A freshly prepared solution of clean sodium (1.21 g, 52 meq) in anhydrous EtOH (20 ml) was added in 3 minutes to a magnetically stirred solution of diethyl allylmalonate (20.0 g, 50 mmole) in sieve-dried pyridine (40 ml) maintained just short of freezing (-40° to -35°) by means of an acetone/solid $CO_2$ bath. A stream of dry $N_2$ was bubbled through the solution at -35° via a fine porosity gas inlet tube for 5 minutes. By means of a Y connector, the $N_2$ was shut off and $FClO_3$ from a cylinder was bubbled through the same gas inlet at a rate not to exceed an internal temperature of -35° for the vigorously exothermic reaction. $FClO_3$ passage was stopped when no further heat evolution was observed (15 minutes; 17 g used). The pale yellow suspension was flushed with $N_2$ for 5 minutes, and was then partitioned between ether (200 ml) and water (60 ml). The ethereal layer was washed with water (2x60 ml) and the combined aqueous solutions were back extracted with ether (100 ml). The second ethereal extract was washed with water (2x20 ml) and was then combined with the main extract, dried ($MgSO_4$), filtered and evaporated. The residue was distilled to give a colorless oil (9.73 g), bp 63°/0.2 mm; $n_D^{22°}$ 1.4192. NMR ($CDCl_3$) $\delta$ 1.31 (t, 6 H, J = 7.0 Hz,

$[CH_3]_2$); 2.92 (dd, 2 H, $J_{HH}$ = 7.0 Hz, $J_{HF}$ = 24.1 Hz, $CH_2$); 4.30 (q, 4 H, J = 7.0 Hz, $[OCH_2]_2$); 5.22 (m, 2 H, =$CH_2$); 5.77 (m, 1 H, =CH-). Anal. calcd. for $C_{10}H_{15}FO_4$: C, 55.04; H, 6.93; F, 8.71. Found: C, 55.02; H, 6.95; F, 8.58.

Step B:

In a similar manner to step B in Example 1, with temperature control (-30°/7 minute addition; 30°/20 minute intermittent cooling; 20°/30 minutes), the product of Step A, diethyl allylfluoromalonate (20.0 g, 0.15 mole) was reduced to 2-allyl-2-fluoropropane-1,3-diol (12.3 g). The crude product, $R_f$ 0.54, (TLC silica GF, hexane/75% EtOAc, $Ce(SO_4)_2$) contained a more polar impurity, $R_f$ 0.40, which was not removed by distillation (bp 60°/0.1 mm). Chromatography over silica (hexane/40% EtOAc) gave pure material in the early fractions (6.35 g total) [A], followed by increasingly contaminated fractions (4.41 g total) [B]. Distillation of a portion of [A] gave a colorless oil, bp 75°/0.15 mm, $n_D^{22°}$ 1.4513. NMR ($CDCl_3$) XL400, δ 2.25 (t, 2 H, J = 6.4 Hz, $[OH]_2$); 2.48 (dddd, 2 H, $J_{HH}$ = 1.2, 1.2 and 7.5 Hz, $J_{HF}$ = 19.3 Hz, $CH_2$); 3.76 (ddd, 2 H, $J_{HH}$ = 6.4 and 12.0 Hz, $J_{HF}$ = 19.2 Hz, $[CH_AO_2]$); 3.78 (ddd, 2 H, $J_{HF}$ = 6.4 and 12.0 Hz, $J_{HF}$ = 16.8 Hz, $[CH_BO]_2$); 5.17 (m, 2 H, =$CH_2$); 5.82 (ddt, 1 H, J = 7.3, 9.7 and 17.0 Hz, =CH-). Anal. calcd. for $C_6H_{11}O_2F$: C, 53.72; H, 8.27; F, 14.16. Found: C, 53.79; H, 8.19; F, 14.00.

Step C:

In a similar manner to step C in Example 1, the product of Step B 2-allyl-2-fluoropropane -1,3- diol (14.0 g) was converted to the 1,3-dioxane (8.02 g), bp 62°/5.5 mm, $n_D^{23°}$ 1.4300. The low yield was due to sudden onset of decomposition on attempted distillation at atmospheric pressure. Rapid cooling, neutralization with aq. $NaHCO_3$ extraction with ether, and chromatography over silica (hexane/20% EtOAc) recovered about half the product which then was smoothly distilled at reduced pressure. NMR ($CDCl_3$) δ 1.40 (s, 3 H, $CH_3$); 1.45 (s, 3 H, $CH_3$); 2.36 (m, 2 H, $CH_2$); 3.79 (dd, 2 H, $J_{HH}$ = 12.8 Hz, $J_{HF}$ = 23.2 Hz, $[CH_AO]_2$); 3.82 (dd, 2 H, $J_{HH}$ = 12.8 Hz, $J_{HF}$ = 16.8 Hz, $[CH_BO]_2$); 5.19 (m, 2 H, =$CH_2$); 5.82 (m, 1 H, =CH-). Anal. calcd. for $C_9H_{15}O_2F$: C, 62.05; H, 8.68; F, 10.91. Found: C. 62.07; H, 8.74; F, 11.10.

Step D:

In a similar manner to step D in Example 1, the product of Step C, 5-allyl-2,2-dimethyl-5-fluoro-1,3-dioxane (7.92 g, 45.4 mmole) was converted to 2,2-dimethyl-5-fluoro-5-(2′-hydroxyethyl)-1,3-dioxane. The crude product was chromatographed over silica gel (hexane/40-75% EtOAc, with extensive tailing) to give single spot material (4.99 g) (TLC, silica GF, EtOAc, $Ce(SO_4)_2$, $R_f$ 0.47) a portion of which was distilled as a colorless oil, bp 85°/0.15 mm, $n_D^{22°}$ 1.4464, which readily crystallized as needles, mp 47-52°. NMR ($CDCl_3$) δ 1.43 (s, 3 H, $CH_3$); 1.46 (s, 3 H, $CH_3$); 1.86 (dt, 2 H, $J_{HH}$ = 6.0 Hz, $J_{HF}$ = 19.7 Hz, $CH_2$); 3.86 (t, 2 H, J = 6.0 Hz, $OCH_2$); 3.88 (dd, 2 H, $J_{HH}$ = 12.5 Hz, $J_{HF}$ = 24.1 Hz, $[CH_AO]_2$); 3.94 (dd, 2 H, $J_{HH}$ = 12.5 Hz, $J_{HF}$ = 16.6 Hz, $[CH_BO]_2$). NMR ($D_2O$) δ 1.45 (s, 3 H, $CH_3$); 1.53 (s, 3 H, $CH_3$); 1.83 (dt, 2 H, $J_{HH}$ = 6.6 Hz, $J_{HF}$ = 21.0 Hz, $CH_2$); 3.76 (t, 2 H, J = 6.6 Hz, $OCH_2$); 3.94 (dd, 2 H, $J_{HH}$ = 13.3 Hz, $J_{HF}$ = 13.7 Hz, $[CH_AO]_2$); 4.12 (dd, 2 H $J_{HH}$ = 13.3 Hz, $J_{HF}$ = 36.0 Hz, $[CH_BO]_2$). Anal. calcd. for $C_8H_{15}FO_3$: C, 53.92; H, 8.48; F, 10.66. Found: C, 53.72; H, 8.45: F, 10.93.

Step E:

In a similar manner to step E in Example 1, the product of Step D, 2,2-dimethyl-5-fluoro-5-(2-hydroxyethyl)-1,3-dioxane (2.00 g, 11.2 mmole) was converted to 2,2-dimethyl-5-fluoro-5-(2-p-toluenesulphonyloxyethyl)-1,3-dioxane as a pale yellow viscous oil (3.92 g). TLC essentially single spot (silica GF, hexane/50% EtOAc, $Ce(SO_4)_2$, $R_f$ 0.59). NMR ($CDCl_3$) δ 1.40 (s, 3 H, $CH_3$); 1.43 (s, 3 H, $CH_3$); 1.97 (dt, 2 H, $J_{HH}$ = 6.2 Hz, $J_{HF}$ = 19.7 Hz, $CH_2$); 2.46 (s, 3 H, $TsCH_3$); 3.79 (dd, 2 H, $J_{HH}$ = 13.0 Hz, $J_{HF}$ = 22.3 Hz, $[CH_AO]_2$); 3.82 (dd, 2 H, $J_{HH}$ = 13.0 Hz, $J_{HF}$ = 19.8 Hz, $[CH_BO]_2$); 4.19 (t, 2 H, J = 6.2 Hz, $OCH_2$); 7.37 and 7.80 (ABq, 4 H, J = 8.2 Hz, Ar).

Step F:

2-amino-6-benzyloxypurine (3.25 g, 13.5 mmol) was alkylated with 2,2-dimethyl-5-fluoro-5-(2-p-toluenesulphonyloxyethyl)-1,3-dioxane (8.73 g, 11.2 mmol). In this case, the original aqueous washings were back extracted with EtOAc which recovered approximately 20% of the calculated weight. Chromatography and crystallization from EtOAc/trace $CH_2Cl_2$ gave colorless prisms (1.69 g), mp 186-188°. TLC single spot, $R_f$ 0.53 (silica GF, $CHCl_3$/MeOH/$H_2O$, 90:10:1, $Ce(SO_4)_2$). UV (MeOH) $\lambda_{max}$ 212 nm ($\epsilon$ 29,620), 249 nm ($\epsilon$ 8,440), 284 nm ($\epsilon$ 9,820). NMR ($CDCl_3$) $\delta$ 1.40 (s, 3 H, $CH_3$); 1.45 (s, 3 H, $CH_3$); 2.19 (dm, 2 H, $J_{HF}$ = 19.8 Hz, $CH_2$); 3.80 (dd, 2 H, $J_{HH}$ = 12.6 Hz, $J_{HF}$ = 24.3 Hz, $[CH_AO]_2$); 3.86 (dd, 2 H, $J_{HH}$ = 12.6 Hz, $J_{HF}$ = 14.6 Hz, $[CH_BO]_2$); 4.22 (m, 2 H, $NCH_2$); 4.87 (s, 2 H, $NH_2$); 5.57 (s, 2 H, $OCH_2$); 7.30-7.53 (m, 5 H, Ar); 7.61 (s, 1 H, $C_8$-H). Anal. calcd. for $C_{20}H_{24}N_5O_3F$: C, 59.84; H, 6.03; F, 4.73; N, 17.45. Found: C, 59.86, H, 6.02; F, 4.93; N, 17.48.

Step G:

In a similar manner to step G in Example 1, the product of Step F,2-amino-6-benzyloxy-9-[2-(2,2-dimethyl-5-fluoro-1,3-dioxan-5-yl)ethyl]-9H-purine (1.38 g, 3.44 mmol) was hydrolysed to 2-amino-1,9-dihydro-9-(3-fluoro-4-hydroxy-3-hydroxymethyl)butyl-6-oxo-6H-purine, also known as 9-[(4-hydroxy-3-fluoro-3-hydroxymethyl)-butyl]guanine, (875 mg), mp 256-275° d. All of it was crystallized from water (45 ml) to give colorless matted needles (771 mg) mp 274-277° d. UV (aq. 0.01 N NaOH) $\lambda_{max}$ 215 nm ($\epsilon$ 19,380), 256 nm ($\epsilon$ 10,550), 268 nm ($\epsilon$ 11,150). NMR ($D_2O$) $\delta$ 2.28 (dt, 2 H, $J_{HH}$ = 7.7 Hz, $J_{HF}$ = 19.3 Hz, $CH_2$); 3.75 (d, 4H, $J_{HF}$ = 19.2 Hz, $[CH_2O]_2$); 4.26 (t, 2 H, J = 7.7 Hz, $NCH_2$); 7.85 (s, 1 H, $C_8$-H). Anal. calcd. for $C_{10}H_{14}FN_5O_3$: C, 44.28; H, 5.20; F, 7.00; , N, 25.82. Found: C, 44.62; H, 5.17; F, 7.10; N, 25.69.

## EXAMPLE 3

### 9-[(3,4-dihydroxy-3-hydroxymethyl)butyl]guanine

Step A:

Freshly cut, clean lithium (219 mg, 31.5 meq.) in five equal pieces was added to a magnetically stirred solution of 2,2-dimethyl-5-(2-hydroxyethyl)-5-methoxy-1,3-dioxane (2.00 g, 10.5 mmole) in anhydrous ethylamine (50 ml) at reflux (17°) under an acetone/solid $CO_2$ condenser topped with a drierite exit tube. Blue coloration spread entirely through the solution in 35 minutes and had discharged completely in 1 hour, 30 minutes. More lithium (146 mg, 21 meq.) was added which permanently restored the blue color. After 4 hours, 40 minutes, the color was discharged by adding dropwise an excess of MeOH (5 ml, violent reaction) which formed a gelatinous mass from which $EtNH_2$ was evaporated (water bath, 35°). The residue was dried at 60°/0.5 mm to give a colorless powder, which was dissolved in water (20 ml) and titrated to pH 8.5 by adding aq. 2N-HCl. The solution was extracted with $CHCl_3$ (4x20 ml). The combined extracts were dried ($MgSO_4$), filtered, and evaporated. The residue was distilled to give a colorless viscous oil (728 mg), bp approx. 115°/0.18 mm, $n_D^{24°}$ 1.4644. NMR ($CDCl_3$) $\delta$ 1.43, (s, 3 H, $CH_3$); 1.45 (s, 3 H, $CH_3$); 1.69 (t, 2 H, J = 5.9 Hz, $CH_2$); 3.67 (d, 2 H, J = 11.4 Hz, $[CH_AO]_2$); 3.80 (d, 2 H, J = 11.4 Hz, $[CH_BO]_2$); 3.83 (t, 2 H, J = 5.9 Hz, $OCH_2$). Anal. calcd. for $C_8H_{16}O_4$: C, 54.53; H, 9.15. Found: C, 54.30; H, 9.14.

Step B:

In a similar manner to step E in example 1, 2, 2-dimethyl-5-hydroxy-5-(2-hydroxyethyl)-1,3-dioxane (533 mg, 3.0 mmole) was converted to 2,2-dimethyl-5-hydroxy-5-(2-p-toluenesulphonyloxyethyl)-1,3-dioxane as a pale orange viscous oil (969 mg). TLC essentially single spot (silica GF, hexane/50% EtOAc, $Ce(SO_4)_2$, $R_f$ 0.49). NMR ($CDCl_3$) $\delta$ 1.42 (s, 6 H, $[CH_3]_2$); 1.73 (t, 2 H, J = 6.2 Hz, $CH_2$); 2.45 (s, 3 H, $TsCH_3$); 3.25 (s, 1 H, OH); 3.52 (d, 2 H, J = 11.9 Hz, $[CH_AO]_2$); 3.81 (d, 2 H, J = 11.9 Hz, $[CH_BO]_2$); 4.22 (t, 2 H, J = 6.2 Hz, $OCH_2$); 7.36 and 7.79 (ABq, 4 H, 8.3 Hz, Ar).

Step 6:

In a similar manner to step F in Example 1, 2, 2-amino-6-benzyloxypurine (803 mg, 3.3 mmol) was alkylated with2,2-dimethyl-5-hydroxy-5-(2-p-toluenesulphonyloxyethyl)-1,3-dioxane (1.0 g, 3.0 mmol). The crude product (783 mg) was purified by reverse phase HPLC using a Whatman Partisil M20 10/50 ODS-3 column; aq. 60% MeOH; 10 ml/min; UV monitor at 260 nm. Five consecutive injections of 150 mg in 2.0 ml aq. 60% MeOH were made interspersed with column regeneration with 100% MeOH. The combined product fractions (49 minute peak) were evaporated at 60°/0.5 mm and the residue was recrystallized twice from EtOH to give 2-amino-6-benzyloxy-9-[2-(2,2-dimethyl-5-hydroxy-1,3-dioxan-5-yl)ethyl]-9H-purine mp 176-178°. TLC single spot $R_f$ 0.49 (silica GF, $CHCl_3/MeOH/H_2O$, 90:10:1, $Ce(SO_4)_2$). UV (MeOH) $\lambda_{max}$ 211 nm ($\epsilon$ 30,530), 249 nm ($\epsilon$ 8,850), 283 nm ($\epsilon$ 10,620). NMR $CDCl_3$) $\delta$ 1.42 (s, 3 H, $CH_3$); 1.44 (s, 3 H, $CH_3$); 1.95 (t, 2 H, J = 7.5 Hz, $CH_2$); 3.54 (d, 2 H, J = 11.4 Hz, $[CH_AO]_2$); 3.75 (d, 2 H, J = 11.4 Hz, $[CH_BO]_2$); 3.76 (s, 1 H, OH); 4.24 (t, 2 H, J = 7.5 Hz, $NCH_2$); 4.87 (br s, 2 H, $NH_2$); 5.58 (s, 2 H, $OCH_2$); 7.37-7.52 (m, 5 H, Ar); 7.63 (s, 1 H, $C_8$-H). Anal calcd. for $C_{20}H_{25}N_5O_4$: C, 60.13; H, 6.31; N, 17.53. Found: C, 60.16; H, 6.34; N, 17.59.

Step D:

In a similar manner to step G in Example 1, the product of Step C, 2-amino-6-benzyloxy-9-[2-(2,2-dimethyl-5-hydroxy-1,3-dioxan-5-yl)ethyl]-9H-purine (1.70 mg, 0.43 mmol) was hydrolysed to dihydro-2-amino-1,9- 9-(3,4-dihydroxy-3-hydroxymethyl)butyl-6-oxo-6H-purine, also known as 9-[(3,4-dihydroxy-3-hydroxymethyl)butyl)guanine, (108 mg) mp 270-282° d. Recrystallization twice from water gave colorless microcrystals (80 mg) mp 287-290° d. UV (aq. 0.01 N NaOH) $\lambda_{max}$ 212 nm ($\epsilon$ 23,170), 255 infl. nm ($\epsilon$ 10,120), 268 nm ($\epsilon$ 10m960). NMR ($D_2O$) $\delta$ 2.06 (t, 2H, J = 8 Hz, $CH_2$); 3.58 (s, 4 H, $[CH_2O]_2$); 4.22 (t, 2H, J = 8 Hz, $NCH_2$); 7.85 (s, 1 H, $C_8$-H). Anal. calcd. for $C_{10}H_{15}N_5O_4$: C, 44.60; H, 5.62; N, 26.01. Found: C, 44.32; H, 5.57; N, 25.74.

## Claims

1. A compound of the formula:

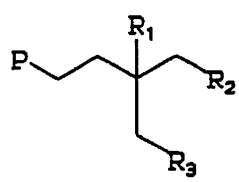

wherein B is a purine, a substituted purine, a pyrimidine or a substituted pyrimidine wherein the substituents on the purine or pyrimidine are selected from amino, hydroxyl, halogen, thio or alkylthiol where in the alkyl moiety of the alkylthiol has 1 to 6 carbons; $R^1$ is fluorine, chlorine, hydroxy, or alkoxy having 1 to 6 carbons or a phosphate group having the formula:

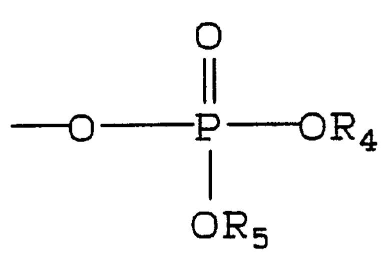

wherein $R^4$ and $R^5$ are hydrogen or pharmaceutically acceptable cations; $R^2$ and $R^3$ are independently hydroxy or alkoxy having 1 to 6 carbons or a phosphate group having the formula:

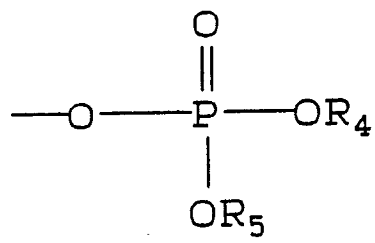

wherein $R^4$ and $R^5$ are hydrogen or pharmaceutically acceptable cations; or two of $R^1$, $R^2$ and $R^3$ taken together form a phosphate group having the formula:

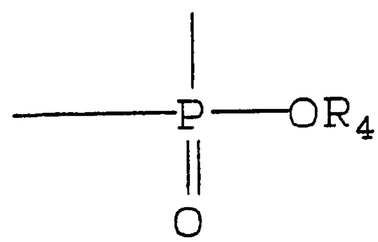

wherein $R^4$ is hydrogen or a pharmaceutically acceptable cation.

2. A compound according to Claim 1 wherein the purine or substituted purine is attached at its 9-position and the pyrimidine or substituted pyrimidine is attached at its 1-position.

3. A compound according to Claim 1 wherein P is guanine, adenine, cytosine, thymine or uracil.

4. 9[(4-hydroxy-3-hydroxymethyl-3-methoxy)butyl]guanine according to Claim 1.

5. 9-[(4-hydroxy-3-fluoro-3-hydroxymethyl)butyl]guanine according to Claim 1.

6. 9-[(3,4-dihydroxy-3-hydroxymethyl)butyl]guanine according to Claim 1.

7. 9-[(4-hydroxy-3-hydroxyethyl-3-methoxy)butyl]-guanine cyclic monophosphate according to Claim 1.

8. 9-[(4-hydroxy-3-fluoro-3-hydroxymethyl)butyl]-guanine cyclic monophosphate according to Claim 1.

9. 9-[(3,4-dihydroxy-3-hydroxymethyl)butyl]-guanine cyclic monophosphate according to Claim 1.

10. An antiviral pharmaceutical composition comprising an effective amount of a compound according to Claim 1 and a pharmaceutically acceptable carrier.

11. The use of a compound according to claim 1, for the preparation of a medicament useful for treating viral infections.